Europäisches Patentamt

European Patent Office

Office européen des brevets

(11) Publication number:

**0 285 569**
**A2**

## EUROPEAN PATENT APPLICATION

(21) Application number: 88810202.7

(22) Date of filing: 28.03.88

(51) Int. Cl.⁴: **C 12 N 1/20**
C 12 N 1/26, C 12 N 1/32

(30) Priority: 30.03.87 JP 74500/87

(43) Date of publication of application:
05.10.88 Bulletin 88/40

(84) Designated Contracting States:
CH DE FR GB LI SE

(71) Applicant: RESEARCH DEVELOPMENT CORPORATION
OF JAPAN
5-2, Nagata-cho 2-chome
Chiyoda-ku Tokyo 100 (JP)

Inoue, Akira
34-B101, Nishiogikita 2-chome Suginami-ku
Tokyo 167 (JP)

Horikoshi, Kouki
39-8 Sakuradai 4-chome Nerima-ku
Tokio 176 (JP)

(72) Inventor: Inoue, Akira
34-B101, Nishiogikita 2-chome
Suginami-ku Tokyo 167 (JP)

Horikoshi, Kouki
39-8, Sakuradai 4-chome
Nerima-ku Tokyo 176 (JP)

(74) Representative: Kerr, Andrew
Postfach 122 Finkelerweg 44
CH-4144 Arlesheim BL (CH)

The microorganism(s) has (have) been deposited with Fermentation Research Institute under number(s) FERM BP-1751, FERM BP-1749 and FERM BP-1750.

(54) **Method for culturing microorganisms.**

(57) A method for culturing microorganisms belonging to the genus Pseudomonas or the genus Escherichia and having tolerance to an organic solvent such as any one of hydrocarbons, alcohols, ethers, ketones and their derivatives or their mixture in a medium containing the organic solvent in a concentration of 0.3% or more. The present method can be widely utilized in the fields of bioreactor, liquid-waste treatment, protein engineering, etc.

EP 0 285 569 A2

**Description**

## METHOD FOR CULTURING MICROORGANISMS

This invention relates to a method for culturing a microorganism which possesses tolerance to organic solvents. In particular, it relates to a method for culturing a microorganism which possesses tolerance to organic solvents and in particular hydrocarbons, alcohols, ethers, ketones and derivatives and mixtures thereof.

It has previously been proposed to culture microorganisms in a medium containing hydrocarbons or their derivatives. It is known for example to culture Nocardia sp. in a medium containing hexane or hexadecane R.L. Raymond, Appl. Microbiol, vol. 15, pp. 857-865 (1967) , to culture Bacterium JOB5 in a medium containing cyclopentane or cyclohexane J. Ooyama and J.W. Foster, Antonie von Leenwenlook, vol. 31, pp. 45-65 (1965) and to culture Pseudomonas sp., Achromobacter sp., and Nocardia sp. in a medium containing benzene, ethyl benzene, toluene or xylene D. Cleus and N. Walkes, J. Gen. Microbiol, vol. 36, pp. 107-122 (1964) . However, in all these earlier cases the microorganisms have been cultured in the presence of hydrocarbons at low concentrations or in the vapour state since hydrocarbons are generally toxic towards microorganisms. That is, when the fermentation is carried out using these hydrocarbons as substrates, it is carried out by supplying these compounds in gaseous form so that the organic compounds do not come directly into contact with the microorganisms, or by keeping the compounds at a low concentration (0.2% or less) such that there is no toxic effect. Consequently, when carrying out fermentations using hydrocarbons as substrates, there are problems not only of low productivity but also of operation because of the difficulty in adjusting the substrates to the low concentration. Furthermore, when using substances having low solubility in water, there is the disadvantage that the productivity becomes low in the microbial reaction owing to the low solubility of the substance.

In order to solve the foregoing problems, the present inventors have extensively studied soil samples to obtain a microorganism which can grow in a medium containing a solvent such as a hydrocarbon or the like in a high concentration, that is, a microroganism having a tolerance to a solvent such as hydrocarbons or the like. As a result, the present inventors have found microorganisms having the aforementioned tolerance and disclose a method for culturing these microorganisms in a medium containing an organic solvent.

It is an object of the present invention to provide a method for culturing a microorganism which displays tolerance to organic solvents.

Because microorganisms belonging to the genus Pseudomonas or the genus Escherichia according to the present invention have excellent tolerance to solvents containing hydrocarbons, alcohols, ethers, ketones and derivatives or mixtures thereof which are commonly used. Saprophyte contamination of said microorganisms can be avoided by culturing the same in the presence of the above solvents. In this case, because heating (pasteurization) is no longer necessary, it becomes possible to use thermolabile additives. When culturing using these solvents as substrates, the substrates can be present in high concentrations. This leads to increased productivity. In addition, if the substrate concentration is high as described above, control over addition of the substrate becomes easier. In the case of toxic substances to be used for the culture by dissolving in these solvents, it also becomes possible to control concentration. Similarly, in the case of slightly water-soluble substances to be used also by dissolving in the solvents, they can be used at high concentrations. The present method for culturing microorganisms can thus be widely employed in the fields of bioreactor, liquid-waste treatment, protein engineering, etc.

According to the present invention therefore there is provided a method for culturing a microorganism which comprises culturing a microorganism belonging to the genus Pseudomonas or the genus Escherichia and having a tolerance to an organic solvent in a medium containing 0.3% or more of the organic solvent.

Microorganisms belonging to the genus Pseudomonas which may be used include Pseudomonas aeruginosa, Pseudomonas fluorescens, Pseudomonas putida, Pseudomonas sp. STM-801 and Pseudomonas sp. STM-904. Microorganisms belonging to the genus Escherichia which can be used include Escherichia coli.

Organic solvents to which these microorganisms show tolerance include for example aliphatic hydrocarbons, alicyclic hydrocarbons, aromatic hydrocarbons, alcohols, ethers, ketones and derivatives an mixtures thereof.

The microorganisms belonging to the genus Pseudomonas or the genus Escherichia can be cultivated in a medium containing aliphatic hydrocarbons, alicyclic hydrocarbons, alcohols, ethers, ketones, aromatic hydrocarbons which are particularly highly toxic and their derivatives in a concentration as high as 0.3% or more. In addition, these strains can grow even in a medium containing the above compounds in a concentration as high as 50% or more. Thus, in the culture of these microorganisms, a substrate can be supplied in large quantities, whereby improvements in productivity and the control over the substrate concentration become easier and minimization or indeed prevention of saprophyte contamination becomes possible. Furthermore, improvements in productivity in the microbial reaction and control over the concentration of toxic substances become possible by dissolving slightly water-soluble substances in various hydrocarbons.

The strains belonging to the genus Pseudomonas according to the present invention, i.e., STM-603, STM-801 and STM-904 were obtained by culturing soil which the present inventors collected from various soil samples, in media containing 0.1% glucose, 0.25% yeast extract, 0.5% peptone and 50% solvent (aliphatic hyorocarbons, alicyclic hydrocarbons, aromatic hydrocarbons, alcohols, ethers and ketones) and then isolating colonies formed.

As specific examples of solvents, pentane, hexane, heptane, octane, isooctane, nonane, decane 1- or 2-hexene, 1-octene, 1-dodecene, 1,3-pentadiene, 1,5-hexadiene, 1,7-octadiene, etc. as aliphatic hydrocarbons; cyclopentane, cyclohexane, methyl cyclopentane, methylcyclohexane, etc. as alicyclic hydorcarbons; toluene, xylene, styrene, ethyl benzene, chlorobenzene, etc. as aromatic hydrocarbons; 1-heptanol, 1-octanol, 1-decanol, etc. as alcohols; n-hexyl ether, n-butyl phenyl ether, diphenyl ether, dibenzyl ether, methoxytoluene, etc. as ethers; and 2-pentanone, 2-hexanone, 2-heptanone, cyclohexanone, etc. as ketones can be enumerated.

These strains, STM-603, STM-801 and STM-904 have the following bacteriological properties:

T a b l e   1

Morphological and Physiological Properties of
Isolates STM-603, STM-801 and STM-904

| | STM-603 | STM-801 | STM-904 |
|---|---|---|---|
| A. Morphological Properties (24-hr. culture in a meat juice liquid medium) | | | |
| a. Shape and Size of Cell | rod | rod | rod |
| | $0.7\sim1.0\mu m \times 2\sim4\mu m$ | $0.7\sim1.0\mu m \times 2\sim15\mu m$ | $0.7\sim1.0\mu m \times 3\sim15\mu m$ |
| b. Polymorphism of Cell | − | + or − | + or − |
| c. Motility | − | − | − |
| d. Sporulation | − | − | − |
| d. Gram's Stain | − | − | − |
| B. Growth on various media (24-hr. culture at 30℃) | | | |
| a. Meat juice agar plate culture | Circular colonies of 0.5 to 1mm with lustrously flesh-colored surface | Circular colonies of 0.5 to 1mm with lustrously flesh-colored surface | Circular colonies of 0.5 to 1mm with lustrously flesh-colored surface |
| b. Meat juice agar slant culture | Grown on the surface of the medium | Grown on the surface of the medium | Grown on the surface of the medium |
| c. Meat juice liquid medium | Grown | Grown | Grown |

| | | | |
|---|---|---|---|
| d. Meat juice gelatin stab culture | No gelatin lique-faction | No gelatin lique-faction | No gelatin lique-faction |
| C. Physiological Properties | | | |
| a. Nitrate reduction | − | − | − |
| b. Starch hydrolysis | − | − | − |
| c. Poly-$\beta$-hydroxybutyrate hydrolysis | − | − | − |
| d. Tween 80 hydrolysis | − | − | − |
| e. Arginine hydrolysis | + | + | + |
| f. Pigment formation (King B medium) | Yellowish green, water-soluble fluorochrome | Yellowish green, water-soluble fluorochrome | Not formed |
| g. Oxydase | + | + | + |
| h. Catalase | + | + | + |
| i. Grwoth range: | | | |
|     pH | 5.0~9.5 | 5.0~9.0 | 5.0~9.0 |
|     Temperature | Not grown at 41℃ | Not grown at 41℃ | Not grown at 41℃ |
| j. Behavior toward oxygenn | Aerobic | Aerobic | Aerobic |
| k. O-F test | Oxidative | Oxidative | Oxidative |
| l. Citrate utilization | + | + | + |

0 285 569

| | | | |
|---|---|---|---|
| m. Levan production from sucrose | − | − | − |
| n. DNase production | − | − | − |
| o. Acylamidase production | − | − | − |
| p. Assimilation: | | | |
|     D-glucose | + | + | + |
|     D-fructose | + | + | + |
|     D-xylose | + | + | + |
|     D-maltose | − | − | − |
|     Sucrose | − | − | − |
|     Lactose | − | − | − |
|     D-trehalose | − | − | − |
|     Mannitol | − | − | − |
|     2-ketogluconic acid | + | + | + |
|     L-valine | + | + | + |
|     β-alanine | + | + | + |
|     DL-arginine | + | + | + |
|     Acetoamido | − | − | − |

| Meso-inositol | Benzyl amine | Geraniol |
|---|---|---|
| – | + | – |
| – | + | – |
| – | + | – |

On the basis of the bacteriological properties given in Table 1, the screening of each strain was carried out according to *Bergey's Manual of Determinative Bacteriology,* (8th ed., 1975). As a result, the bacteriological properties of the strain STM-603 were compatible with those of Pseudomonas putida. However, Pseudomonas putida does not tolerate hydrocarbons. The tolerance of IFO 3738 as a standard strain of Pseudomonas putida and the present strain STM-603 to various solvents were examined. The results were given in Table 2.

T a b l e  2

| Solvent | Pseudomonas putida IFO 3738 | STM-603 |
|---------|----------------------------|---------|
| Toluene | — | + |
| p- Xylene | — | + |
| Styrene | — | + |

Since the strain STM-603 and Pseudomonas putida had morphological, physiological and bacteriological properties in common but differed from one another as regards solvent tolerance, the strain STM-603 was recognized as a new strain belonging to Pseudomonas putida and designated as Pseudomonas putida var. STM 603.

The strains STM-801 and STM-904 are basically similar to Pseudomonas putida.

Thus, the present inventors have made a further detailed comparisonsbetween the present strains STM 801 and STM-904 and IFO 3738 as a standard strain of Pseudomonas putida with respect to their bacteriological properties, whereby obtaining the following results, as will be also given in Table 3:

(1) The cell size of Pseudomonas putida is 0.7 to 1.0µ by 2 to 4µ, while that of STM-801 and STM-904 are respectively 0.7 to 1.0µ by 2 to 15µ and 0.7 to 1.0µ by 3 to 15µ. That is, the size of the present strains is 3 to 4 times that of Pseudomonas putida.

(2) Regarding motility, all cells show motility in the case of Pseudomonas putida. However, in the case of STM-801 and STM-904, some cells show motility, whilst others do not.

(3) Regarding pigment formation, yellowish green water-soluble fluorochrome is formed in the case of Pseudomonas putida. However, in the case of STM-801 and STM 904, the former forms the same pigment as above, but the latter does not.

(4) Regarding solvent tolerance with respect to toluene, p-xylene and styrene, Pseudomonas putida shows no tolerance at all while STM-801 and STM-904 show tolerance.

From these results, it is readily apparent that the strains STM 801 and STM-904 are new species because they are clearly different from Pseudomonas putida and becuase there is no known species corresponding to these strains. So, the present inventors designated the strains STM 801 and STM 904 as Pseudomonas sp. STM 801 and Pseudomonas sp. STM-904 respectively.

Table 3

| | Pseudomonas putida IFO 3738 | STM-801 | STM-904 |
|---|---|---|---|
| (1) Size of Cell | 0.7~1.0X2~4μ | 0.7~1.0X2~15μ | 0.7~1.0X3~15μ |
| (2) Motility | + | + or − | + or − |
| (3) Pigment Formation | Yellowish green, water-soluble fluorochrome | Yellowish green, water-soluble fluorochrome | Not formed |
| (4) Solvent Tolerance: | | | |
| Toluene | − | + | + |
| P-xylene | − | + | + |
| Styrene | − | + | + |

Said Pseudomonas putida var. STM 603, Pseudomonas sp. STM 801 and Pseudomonas sp. STM 904 were deposited in Fermentation Research Institute of Agency of Industrial Science and Technology respectively with the accession numbers FERM BP- 1751 (Bikoken-kinki No. 9228), FERM-BP1749 (Bikoken-kinki No. 9226) and FERM BP 1750 (Bikoken-kinki No. 9227).

As the present microorganisms belonging to the genus Pseudomonas other than the aforementioned strains, Pseudomonas aeruginosa IFO-3924, Pseudomonas fluorescens IFO-3507, Pseudomonas putida IFO-3738, etc. can be enumerated, and as the present microorganism belonging to the genus Escherichia, Escherichia coli IFO-3806 can be enumerated.

As a medium for culturing these strains, an ordinary medium containing a carbon source, a nitrogen source, an inorganic ion, etc. is used.

As a carbon source, any of those which can be assimilated, for example, sugars such as glucose, fructose, xylose, starch hydrolysate, etc., hydrocarbons such as toluene, p-xylene, etc., alcohols such as methanol, ethanol, etc., etc. can be used. As a nitrogen source, yeast extract, dry yeast, peptone, meat extract, corn steep liquor, casamino acid, ammonium chloride, ammonium sulfate, urea, sodium nitrate, etc. are used. As an inorganic ion, phosphoric acid ion, magnesium ion, iron ion, calcium ion, potassium ion, copper ion, manganese ion, etc. are used.

As solvents, aliphatic hydrocarbons, alicyclic hydrocarbons, aromatic hydrocarbons, alcohols, ethers, ketones and their derivatives or their mixture can be used. As specific examples of these solvents, pentane, hexane, heptane, octane, isooctane, nonane, decane, 1- or 2-hexene, 1-octene, 1-dodecene, 1,3-pentadiene, 1,5-hexadiene, 1,7-octadiene, etc. as aliphatic hydrocarbons; cyclopentane, cyclohexane, methyl cyclopentane, methyl cyclohexane, etc. as alicyclic hydrocarbons; toluene, xylene, styrene, ethyl benzene, chlorobenzene, etc. as aromatic hydrocarbons; 1-heptanol, 1-octanol, 1 decanol, etc. as alcohols; n-hexyl ether, n-butyl phenyl ether, diphenyl ehter, dibenzyl ether, methoxytoluene, etc. as ethers; and 2-pentanone, 2-hexanone, 2-heptanone, cyclohexanone, etc. as ketones can be enumerated. The contents of these solvent in a medium is 0.3% or more. Said solvents may be either contained in a medium previously or added to a medium afterwards.

The culture is carried out at pH 5 to 9 at 20 to 40°C under aerobic conditions.

Test Example 1

Pseudomonas putida var. STM-603, Pseudomonas sp. STM 801, Pseudomonas sp: STM 904 and various known strains were respectively inoculated into media (pH 7.0) respective ly containing 1.0g/$\ell$ of glucose, 2.5g/$\ell$ of yeast extract and 5.0g/$\ell$ of peptone. Then, each 5 ml of solvent given in Table 4 was added to each 5 ml of said media. After culturing the resulting media at 37°C for 48 hours, the growth of each strain was compared. The results were given in Table 4.

T a b l e  4  <u>Comparison of Solvent Tolerance of Various Strains</u>

| Strain | Cyclohexane | Toluene | p-Xylene | Styrene |
|---|---|---|---|---|
| Pseudomonas putida var. STM-603 | + | + | + | + |
| Pseudomonas sp. STM-801 | + | + | + | + |
| Pseudomonas sp. STM-904 | + | + | + | + |
| Pseudomonas fluorescens IFO-3507 | − | − | − | − |
| Pseudomonas pseudoolcaligenes ATCC-12815 | − | − | − | − |
| Arthrobacter globiformus IFO-3062 | − | − | − | − |
| Agrobacterium tumefaciens IFO-3058 | − | − | − | − |
| Escherichia coli IFO-3806 | − | − | − | − |
| Bacillus cereus IFO-3131 | − | − | − | − |
| Bacillus coagulans IFO-3557 | − | − | − | − |

−:  Not grown    +:  Grown $(O.D_{660} > 0.50)$

Example 1

A medium prepared by adding 1.0ℓ of distilled water to 1.0g of glucose, 2.5g of yeast extract and 5.0g of peptone and adjusted to pH 7.2 was dispensed in 100mℓ portions into 500-mℓ ribbed conical flasks, in which Pseudomonas putida var. STM 603 was inoculated without sterilizing said flasks. After adding each 30mℓ of toluene to the flasks, the culture was carried out at 37°C for 48 hours. As a result 1.2mg/mℓ of Pseudomonas putida var. BTM-603 cell mass was obtained, where the contamination and the growth of other microorganisms were not observed.

Example 2

A medium prepared by adding 1.0ℓ of distilled water to 1.0g of glucose. 2.5g of yeast extract and 5.0g of peptone and adjusted to pH 7.2 was dispensed in 100mℓ portions into 500-mℓ ribbed conical flasks, in which Pseudomonas sp. STM-801 was inoculated without sterilizing said flasks. After adding each 30mℓ of toluene to the flasks, the culture was carried out at 37°C for 48 hours. As a result, 1.1mg/mℓ of Pseudomonas sp. STM-801 cell mass was obtained, where the contamination and the growth of other microorganisms were not observed.

Example 3

Strains of the genus Pseudomonas were inoculated in each 5mℓ of sterile meat juice liquid medium (containing 5.0g of meat extract, 15.0g of peptone, 5.0g of sodium chloride, 5.0g of dibasic potassium phosphate and 1ℓ of distilled water; pH 7.0), and a strain of the genus Escherichia was inoculated in 5mℓ of sterile LB liquid medium (containing 10g of tryptone, 5g of yeast extract, 10g of sodium chloride, 1g of glucose and 1ℓ of distilled water pH 7.0). Then, each 5mℓ of solvents given in Table 5 was added to each medium. The culture was carried out at 30°C for 48 hours. The state of the growth of each strain was given in Table 5. The growth was monitored by measuring turbidity (wave length: 660nm, a colorimeter "Spectronic 21" manufactured by Bausch &; Lomb Corp.)

Table 5

| Strain | Solvent | |
| --- | --- | --- |
| | hexane | cyclohexane |
| Pseudomonas aeruginosa IFO 3924 | − | + |
| Pseudomonas fluorescens IFO 3507 | + | − |
| Pseudomonas putids IFO 3738 | + | + |
| Escherichia coli IFO 3806 | + | − |

−: Not grown    +: Grown ($O.D_{660} > 0.50$)

Example 4

The same medium as that of Example 1 was prepared, dispensed in 5mℓ portions into large test tubes and steam-sterilized at 121°C for 15 minutes. Then, Pseudomonas putida var. STM-603, Pseudomonas sp. STM-801 and Pseudomonas sp. STM 904 were respectively inoculated in the large test tubes, to which each 5mℓ of various solvents given in Table 6 was added. The culture was carried out at 37°C using a test tube shaker. The state of growth after 48 hours was given in Table 6. The growth was monitored by measuring turbidity (wave length: 660nm, a colorimeter "Spectronic 21" manufactured by Bausch &; Lomb Corp.).

T a b l e  6

Tolerance of Pseudomonas putida var. STM-603,
Pseudomonas sp. STM-801 and Pseudomonas sp.
STM-904 to Various Solvents

| Solvent | Strain | | |
|---|---|---|---|
| | STM-603 | STM-801 | STM-904 |
| Aliphatic hydrocarbons: | | | |
| pentane | + | + | + |
| hexane | + | + | + |
| heptane | + | + | + |
| octane | + | + | + |
| isooctane | + | + | + |
| nonane | + | + | + |
| decane | + | + | + |

| | | | |
|---|---|---|---|
| 1- or 2-hexene | + | + | + |
| 1-octene | +. | + | + |
| 1-dodecene | + | + | + |
| 1,3-pentadiene | ± | ± | ± |
| 1,5-hexadiene | + | + | + |
| 1,7-octadiene | + | + | + |

Alicyclic hydrocarbons:

| | | | |
|---|---|---|---|
| cyclopentane | + | + | + |
| methyl cyclopentane | + | + | + |
| cyclohexane | + | + | + |
| methyl cyclohexane | + | + | + |
| butyl cyclohexane | + | + | + |
| cyclooctane | + | + | + |

Aromatic hydrocarbons:

| | | | |
|---|---|---|---|
| toluene | + | + | + |
| p-xylene | + | + | + |
| o-,m-p-xylene | + | + | + |
| chlorobnezene | + | + | + |
| o-dichlorobenzene | + | + | + |
| 1,2,4-trichlorobenzene | + | + | + |
| bromobenzene | + | + | + |
| ethyl benzene | + | + | + |
| propyl benzene | + | + | + |
| styrene | + | + | + |

0 285 569

Alcohols:

| | | | |
|---|---|---|---|
| 1-heptanol | + | + | + |
| 1-octanol | + | + | + |
| 1-decanol | + | + | + |

Ethers:

| | | | |
|---|---|---|---|
| n-hexyl ether | + | + | + |
| n-butyl phenyl ether | + | + | + |
| diphenyl ether | + | + | + |
| dibenzyl ether | + | + | + |
| methoxytoluene | + | + | + |

$\pm$: Grown $(0 < O.D_{660} < 0.50)$

$+$: Grown $(O.D_{660} > 0.50)$

Example 5

The same medium as that of Example 1 was prepared, dispensed in 5mℓ portions into large test tubes and steam-sterilized at 121°C for 15 minutes. Then, Pseudomonas putida STM-603, Pseudomonas sp. STM 801 and Pseudomonas sp. STM 904 were respctively inoculated in the test tubes, to each of which each 0.25mℓ of various solvents given in Table 7 was added. The culture was carried out at 37°C using a test tube shaker. The state of growth after 48 hours was given in Table 7. The growth was monitored by measuring turbidity (wave length: 660nm, a colorimeter "Spectronic 21" manufac tured by Bausch &; Lomb Corp.).

15

Table 7

| Solvent | Strain | | |
|---|---|---|---|
| Ketones | STM-603 | STM-801 | STM-904 |
| 2-pentanone | ± | ± | ± |
| 2-hexanone | ± | ± | ± |
| 2-heptanone | + | + | + |
| cyclohexanone | ± | ± | ± |

−: Not grown

±: Grown ($0 < O.D_{660} < 0.50$)

+: Grown ($O.D_{660} > 0.50$)

## Claims

1. A method of culturing a microorganism, which comprises culturing a microorganism belonging to the genus Pseudomonas or the genus Escherichia said microorganism having a tolerance to an organic solvent in a medium containing 0.3% or more of the organic solvent.

2. A method according to Claim 1, wherein the microorganism belonging to the genus Pseudomonas is Pseudomonas aeruginosa, Pseudomonas fluorescens, Pseudomonas putida, Pseudomonas sp. STM-801 or Pseudomonas sp. STM-904.

3. A method according to Claim 2, wherein the Pseudomonas aeruginosa is Pseudomonas aeruginosa IFO-3924.

4. A method according to Claim 2, wherein the Pseudomonas fluorescens is Pseudomonas fluorescens IFO-3507.

5. A method according to Claim 2, wherein the Pseudomonas putida is Pseudomonas putida var. STM-603 or Pseudomonas putida IFO-3738.

6. A method according to Claim 1, wherein the microorganism belonging to the genus Escherichia is Escherichia coli.

7. A method according to Claim 6, wherein the Escherichia coli is Escherichia coli IFO-3806.

8. A method according to Claim 1, wherein the culture medium containing 0.3% or more organic solvent is prepared by adding 0.3% or more of an organic solvent to a medium containing no organic solvent.

9. A method according to Claim 1 or 8, wherein the organic solvent is an aliphatic hydrocarbon, alicyclic hydrocarbon, aromatic hydrocarbon, alcohol, ether, ketone or a derivative of mixture thereof.

10. A method of culturing a microorganism as claimed in any one of Claims 1 to 9 wherein said microorganism can maintain growth in a medium wherein said one or more of aliphatic hydrocarbons, alicyclic hydrocarbons, aromatic hydrocarbons, alcohols, ethers, ketones, and their derivatives or their mixtures is present in a concentrataion of 0.3% or more.

11. A method of culturing a microorganism as claimed in any one of Claims 1 to 9, wherein said microorganism can maintain growth in a medium wherein said one or more of aliphatic hydrocarbons, alicyclic hydrocarbons, aromatic hydrocarbons, alcohols, ethers, ketones, and their derivatives or their mixtures is present in a concentration of up to 50% or more.

12. A method of culturing a microorganism as claimed in any one of Claims 1 to 9 wherein said microorganism can maintain growth in a medium wherein said one or more of aliphatic hydrocarbons, alicyclic hydrocarbons, aromatic hydrocarbons, alcohols, ethers, ketones, and their derivatives or their mixtures is present in a concentration of more than 50%.

13. A method of culturing a microorganism as claimed in any one of Claims 1 to 12 wherein in said one or more of aliphatic hydrocarbons, alicyclic hydrocarbons, aromatic hydrocarbons, alcohols, ethers, ketones and their derivatives or their mixtures is pentane, hexane, heptane, octane, isooctane, nonane, decane, 1- or 2-hexene, 1-octene, 1-duodecene, 1,3-pentadiene, 1,5-hexadiene, 1,7-octadiene, cyclopentane, cyclohexane, methyl cyclopentane, methyl cyclohexane, toluene, xylene, styrene, ethyl benzene, chlorobenzene, 1-heptanol, 1-octanol, 1-decanol, n-hexyl ether, n-butyl phenyl ether, diphenyl ether, dibenzyl ether, methoxytoluene, 2-pentanone, 2-hexanone, 2-heptanone or cyclohexanone.

14. A method of culturing a microorganism wherein said method is a bioreactor method, a liquid-water treatment or involves protein engineering.

15. Products whenever produced by a process as claimed in claim 14.

17